# EUROPEAN PATENT SPECIFICATION

(11) **EP 2 901 992 B1**
(45) Date of publication and mention of the grant of the patent: **28.12.2016**
(21) Application number: 14153470.1
(22) Date of filing: 31.01.2014
(51) Int. Cl.: A61F 13/534, A61F 13/535, A61F 13/537, A61F 13/53, A61F 13/539, A61F 13/531

(54) **MULTI-LAYER ABSORBENT MATERIAL**
MEHRSCHICHTIGES ABSORBIERENDES MATERIAL
MATÉRIAU ABSORBANT MULTICOUCHE

(43) Date of publication of application: 05.08.2015
(73) Proprietor: Ontex BVBA, 9255 Buggenhout (BE)
(72) Inventor: WEBER, Ainas, 56727 Mayen (DE); DE POORTER, Annick, 9320 Erembodegem - Aalst (BE)
(74) Representative: Brantsandpatents bvba

(56) References cited:
- US-A- 5 865 824
- US-A1- 2001 014 797
- US-A1- 2010 280 479
- US-A1- 2011 270 204
- US-A1- 2012 203 191
- US-A1- 2014 005 622

## Description

### TECHNICAL FIELD

The present invention relates to a multi-layer absorbent material. More particularly, the invention relates to a layered, composite absorbent material with individual layers which are constructed and arranged to selectively cooperate to provide desired performance parameters in the composite, layered structure, to methods for manufacturing said multi-layer absorbent material, as well as to the use of a multi-layer absorbent material according to the invention in an absorbent article, particularly a diaper. Such a multi-layer material provides improved ability to absorb and retain fluids and consequently, prevents excessive rewetting and leakage. The present invention is of particular importance to the field of disposable hygiene products, in particular diapers.

### BACKGROUND

Performance objectives of disposable absorbent articles, such as infant diapers, include no product leakage, dry feel to the wearer, and a comfortable fit throughout the product life. Accordingly, absorbent articles typically contain an absorbent core to provide liquid handling and other absorbent functionalities required to meet the product performance objectives. Absorbent cores of absorbent articles are commonly composed of wood pulp fibers, and superabsorbent polymer particles are often distributed in the absorbent cores to enhance the liquid absorbent capacity. Absorbent articles frequently leak before the liquid absorbent capacity of the entire absorbent core is fully utilized. One problem resulting in leakage is the inability of the absorbent core to fully uptake liquids rapidly and completely when large amounts of liquids are discharged into the absorbent article. Another associated problem contributing to leakage is the inability of the absorbent core to move or distribute sufficient amounts of liquid between discharges from a target area portion of the absorbent article to more distal and more remote end regions of the absorbent core which have not been utilized. This results in saturation of only the central target area of the absorbent core and excessive thickness, bulkiness, and sagging of the wet, heavy absorbent material resulting in poor performance, product fit and wearer discomfort. US 6,437,214B1 disclosed an absorbent article which includes an absorbent core having multiple absorbent layers; the intake capability of the absorbent system is maintained by keeping a second layer of the absorbent system at low saturation. A problem with such an absorbent article is that incoming fluids are not properly absorbed and do not provide enough control of the incoming fluids to stop leakage from occurring.
The absorbent core of current absorbent articles does not adequately meet current performance objectives. Consequently, there remains a need for absorbent structures which can provide improved fluid uptake of liquids and improved liquid distribution to move liquids out of the target area to maintain this desirable liquid uptake behavior for the life of the product.

Document US 2010/280479 A1 discloses an absorbent article, comprising a substantially fluid impervious backsheet, a substantially fluid permeable topsheet, an acquisition layer for collecting and distributing fluid, a top core for absorbing fluid, a bottom core for absorbing fluid, elastic members. The acquisition layer, the top core and the bottom core are arranged between the topsheet and the backsheet. The acquisition layer is arranged between the topsheet and the top core, and the bottom core is arranged between the backsheet and the top core. The absorbent article extends along a longitudinal axis from its front end towards its rear end. It has longitudinal side edges extending along the longitudinal axis. The elastic members are arranged adjacent to at least a portion of each longitudinal side edge and each elastic member has a tension in a direction along the longitudinal side edge.

Document US 5 865 824 A discloses an absorbent structure in which an initially flat, dense structure becomes a three-dimensional, high bulk, channeled structure upon wetting. The structure offers unusually high directionality in fluid transport to improve the distribution of fluid in longitudinal articles. The self-bulking of the wetted article can also lead to improved fit in articles such as diapers and in general increases the void volume of the wetted article for high absorbent capacity.

Document US 2014/005622 A1 discloses an absorbent article including a wearer-facing topsheet, a backsheet and an absorbent core disposed between the topsheet and the backsheet, characterized in that the absorbent core includes: a first absorbent layer comprising a first substrate, a layer of first superabsorbent polymer particles deposited on the first substrate, and a fibrous layer of thermoplastic adhesive material covering the layer of first superabsorbent polymer particles; a second absorbent layer, the second absorbent layer comprising a second substrate and a mixed layer deposited on the second substrate, the mixed layer comprising a mixture of second superabsorbent particles and cellulosic fibers, the first absorbent layer and the second absorbent layer being combined together such that at least a portion of the fibrous layer of thermoplastic adhesive material of the first absorbent layer contacts at least a portion of the mixed layer of the second absorbent layer, and wherein the first absorbent layer is placed closer to the topsheet than the second layer.

Document US 2001/014797 A1 discloses an absorbent incontinence pad with a liquid impervious air permeable back sheet and an absorbent unit partly covered by the back sheet, wherein the absorbent unit has a non-woven fabric substrate, an absorbent zone formed by a plurality of highly absorbent layers extending in the form of bands on the surface of the non-woven fabric substrate and an air permeable zone where no such highly absorbent layer exists, which has sufficiently adequate properties to meet incontinence requirements and provides a comfortable feeling during use.

Document US 2012/203191 A1 discloses a water-absorbent sheet comprising a structure in which an absorbent layer containing a water-absorbent resin and an adhesive is sandwiched with nonwoven fabrics from an upper side and a lower side of the absorbent layer, wherein the water-absorbent sheet has a laminate structure in which the absorbent layer is fractionated into a primary absorbent layer and a secondary absorbent layer with a breathable fractionating layer, and wherein at least one sheet out of the nonwoven fabrics is a spunbond nonwoven fabric, and at least a nonwoven fabric at a lower side out of the nonwoven fabrics is a hydrophilic nonwoven fabric. The water-absorbent sheet of the present invention exhibits some effects that a water-absorbent sheet is capable of accomplishing avoidance of gel blocking phenomenon and liquid leakage, while obtaining basic properties as a water-absorbent sheet at a high level, by using specified nonwoven fabrics, and further providing a hydrophilic nonwoven fabric as a lower side of a nonwoven fabric of the absorbent layer of a water-absorbent sheet, even for a water-absorbent sheet containing a very small amount of pulps and being thinner than a conventional product.

Document US 2011/270204 A1 discloses a water-absorbent sheet composition containing a structure in which an absorbent layer containing a water-absorbent resin and an adhesive is sandwiched with two or more sheets of hydrophilic nonwoven fabrics, wherein the water-absorbent sheet composition has a structure in which the absorbent layer is separated in divided parts of a primary absorbent layer and a secondary absorbent layer with a water-permeable substrate having a water permeability index of from 20 to 90, and wherein the water-absorbent resin is contained in the absorbent layer in an amount of from 100 to 1000 g/m2. The water-absorbent sheet composition of the present invention exhibits some excellent effects that the water-absorbent sheet composition is capable of accomplishing thinning and avoidance of gel blocking phenomenon and liquid leakage, while obtaining basic properties as a water-absorbent sheet composition at a high level, even for a water-absorbent sheet composition containing a very small amount of pulps.

The present invention aims to resolve at least some of the problems mentioned above.

The invention thereto aims to provide an improved absorbent article and process for making such an article.

### SUMMARY OF THE INVENTION

The present invention concerns an absorbent article, preferably a disposable absorbent article, such as a diaper. In a first aspect, the present invention specifically concerns a multi-layer absorbent material comprising: a topsheet (23), a backsheet (5), a central absorbent layer (18) positioned between the topsheet (23) and the backsheet (5), said central absorbent layer (18) comprising superabsorbent polymer particles (28), an acquisition and distribution layer (6) positioned between the topsheet (23) and the central absorbent layer (18) for receiving fluids from the topsheet (23) and transferring the fluids for absorption by the central absorbent layer (18), and a booster absorbent layer (26) positioned between the acquisition and distribution layer (6) and the central absorbent layer (18), the booster absorbent layer (26) comprising a nonwoven carrier (2) with superabsorbent polymer particles (3) whereby the booster absorbent layer is comprising a higher concentration of superabsorbent polymer particles than the central absorbent layer. The differential concentration of superabsorbent polymer particles between the two absorbent layers presents the advantage that the incoming fluids can be efficiently absorbed in a twofold way, firstly by the superabsorbent polymer particles (3) in said booster absorbent layer (26) and secondly by the superabsorbent polymer particles (28) in said central absorbent layer (18). The booster absorbent layer comprises a smaller surface area than the central absorbent layer, more preferably the booster absorbent layer comprises a surface area which is about the surface area of the ADL, preferably the booster absorbent layer and the ADL comprise surface areas which differ within about 50%, more preferably within 60%, still more preferably within 70%, even more preferably within 80%, yet more preferably within 90%, still even more preferably within 95% from one another.

A multi-layer absorbent material according to the invention provides an at least three-layered structure with very different properties. The role of the acquisition and distribution layer (6) is to better distribute the fluids, before accessing the underneath booster absorbent layer (26). The booster absorbent layer (26) is comprising superabsorbent polymer particles (3) that are immobilized in a nonwoven carrier (2) comprising a top layer (17) consisting of staple fibers (31) penetrable by the superabsorbent polymer particles (3) and a nonwoven bottom layer (25) hydroentangled to the top layer (17). Said booster absorbent layer (26) provides an increased absorption capacity within the "hot" zone of a diaper, i.e. the zone in a diaper where insults are most likely to occur. To some extent, the fluids are also distributed by that layer, via the capillary voids present between e.g. carrier fibers and SAP particles. Therefore, in a preferred embodiment, the booster layer comprises high-permeability SAPs. Such SAPs maintain the voids, even if the particles are swollen by the fluid.

In a second aspect, the present invention provides an absorbent article comprising a multi-layer absorbent material according to an embodiment of the invention.

In a third aspect, the invention also provides a process for manufacturing a multi-layer absorbent material according to an embodiment of the invention, comprising the steps of: providing a top sheet (23) and a back sheet (5), providing an acquisition and distribution layer (6), providing an absorbent multi-layer core by providing a booster absorbent layer (26) and a central absorbent layer (18), and disposing the absorbent multi-layer core at least partially between the top sheet (23) and the back sheet (5), whereby the booster absorbent layer (26) is disposed between the acquisition and distribution layer (6) and the central absorbent layer (18), and preferably the ADL is disposed between the topsheet and the booster absorbent layer.

To further improve the desired balance of absorbent properties, there have been identified a number of important factors which can allow the absorbent layer regions to better work in combination, and thereby provide an improved overall system performance. The booster absorbent layer (26) works in tandem with the underneath central absorbent layer (18) because each of those layers comprises an amount of superabsorbent polymer particles (3 and 28) which is preferably between 1 g and 20 g, more preferably between 2 g and 15 g, still more preferably between 3 g and 10 g, e.g. 4 g, 5 g, 6 g, 7 g, 8 g, 9 g or any value there between such as about 6.5 g. In a preferred embodiment, the amount of SAP in said booster absorbent layer and the amount of SAP in said central absorbent layer differ by less than 50%, preferably by less than 40%, still more preferably by less than 30%, yet more preferably by less than 20%, even more preferably by less than 10%, most preferably said amounts of SAP are about the same.

The differential concentration of the superabsorbent polymer particles (3 and 28) within the two absorbent layers allows a better absorption of the incoming fluids and the superabsorbent polymer particles (3 and 28) are in a sufficient amount to absorb the incoming fluids.

Furthermore, the acquisition and distribution layer (6) and the booster absorbent layer (26) are placed towards the front edge of the central absorbent layer (18) to better distribute and absorb fluids in the frontal region of an absorbent article. The frontal region is the intake region of incoming fluids and allows an improved fluids intake function.

In a final aspect, the invention provides use of a multi-layer absorbent material according to an embodiment of the invention for manufacturing an absorbent article. The multi-layer absorbent material provided by the invention can be particularly advantageous for use in an absorbent article such as a diaper.

Preferred embodiments are as specified in the dependent claims.

### BRIEF DESCRIPTION OF THE DRAWINGS

The invention will be further described in detail with reference to the figures, in which
**FIG. 1** shows a general schematic cross-sectional view of the multi-layer structure of the absorbent material with a fluffless central absorbent layer (18) according to the present invention.
**FIG. 2** shows a general schematic cross-sectional view of a multi-layer structure of an absorbent material with a central absorbent layer (18) comprising fluff pulp and superabsorbent, which is not part of the present invention.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

As used herein, the following terms have the following meanings:
"Absorbent article" refers to devices that absorb and contain liquid, and more specifically, refers to devices that are placed against or in proximity to the body of the wearer to absorb and contain the various exudates discharged from the body.

Absorbent articles include but are not limited to diapers, adult incontinence briefs, training pants, diaper holders and liners, sanitary napkins and the like. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn.

The absorbent article of the present invention preferably comprises a liquid permeable topsheet, a liquid impermeable back sheet, and an absorbent medium disposed between the topsheet and the backsheet. The absorbent medium may comprise a booster absorbent layer and a central absorbent layer according to the present invention. The absorbent article may also include one or more features such as, but not limited to, ears or side panels, leg cuffs, fastener components and/or a belt. The top sheet, back sheet and the absorbent medium could be made from any suitable material known to the person skilled in the art.

"Acquisition and distribution layer" or "ADL", also refers to a sub-layer which preferably is a nonwoven wicking layer under the topsheet (or face fabric) of an absorbent product, which speeds up the transport and improves distribution of fluids throughout the absorbent core.

"Absorbent medium" is the absorbent structure disposed between the topsheet and the backsheet of the absorbent article in at least the crotch region of the absorbent article. The absorbent material can be of any conventional kind. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material. Superabsorbent polymers are water-swellable, water-insoluble organic or inorganic materials capable of absorbing at least about 20 times their own weight of an aqueous solution containing 0.9 weight percent of sodium chloride. Organic materials suitable for use as superabsorbent materials can include natural materials such as polysaccharides, polypeptides and the like, as well as synthetic materials such as synthetic hydrogel polymers. Such hydrogel polymers include, for example, alkali metal salts of polyacrylic acids, polyacrylamides, polyvinyl alcohol, polyacrylates, polyvinyl pyridines, and the like. Other suitable polymers include hydrolyzed acrylonitrile grafted starch, acrylic acid grafted starch, and isobutylene maleic anhydride copolymers and mixtures thereof. The hydrogel polymers are preferably lightly cross-linked to render the material substantially water insoluble. Preferred superabsorbent materials are further surface cross-linked so that the outer surface or shell of the superabsorbent particle, fibre, flake, sphere, etc. possesses a higher crosslink density than the inner portion of the superabsorbent. The superabsorbent materials may be in any form which is suitable for use in absorbent composites including particles, fibres, flakes, spheres, and the like.
Preferably, the absorbent medium comprises a nonwoven carrier according to the present invention.

"Diaper" refers to an absorbent article generally worn by infants and incontinent persons about the lower torso.

"Disposable" is used herein to describe articles that are generally not intended to be laundered or otherwise restored or reused (i. e. they are intended to be discarded after a single use and, preferably, to be recycled, composted or otherwise disposed of in an environmentally compatible manner).

"Hydroentanglement process" refers to the manufacturing of nonwoven webs. The process involves directing a series of water jets towards a fibrous web which is supported on a moving porous belt. The water jets pass downwards through the mass of fibres and on making contact with the surface of the belt, the jets rebound, and break up: the energy released causes entanglement of the mass of fibres.

"Nonwoven" refers to a manufactured sheet, web, or batt of directionally or randomly oriented fibres bonded by friction and/or cohesion and/or adhesion, excluding paper and products that are woven, knitted, tufted stitchbonded incorporating binding yarns or filaments, or felted by wet-milling, whether or not additionally needled. The fibres may be of natural or man-made origin. They may also be staple or continuous filaments or be formed in situ.

"Pulp fluff" or "fluff pulp" refers to a material made up of cellulose fibers. The fibers can be either natural or synthetic, or a combination thereof. The material is typically lightweight and has absorbent properties.

"Staple fibers" refer to commercially available fibers having diameters ranging from less than about 0.001 mm to more than about 0.2 mm; they come in several different forms such as short fibers ranging from about 10 to 50 mm in length and long fibers with a length higher than 50 mm, preferably up to 100 mm. The staple fibers for use in the invention can be prepared from thin and/or coarse fibers, preferably polyester fibers. The thin fibers have a dtex below 3, preferably in the range of 1 to 3 whereas the coarse fibers have a dtex of at least 3 and preferably below 45 dtex. In case a blend of thin and coarse fibers is used, the coarse fibres preferably have a dtex value which is at least two times, but no greater than 15 times that of the thin fibers.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

"Topsheet" refers to a liquid permeable material sheet forming the inner cover of the absorbent article and which in use is placed in direct contact with the skin of the wearer. The topsheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. Further examples of topsheet materials are porous foams, apertured plastic films, laminates of nonwoven materials and apertured plastic films etc. The materials suited as topsheet materials should be soft and non-irritating to the skin and be readily penetrated by body fluid, e.g. urine or menstrual fluid. The inner coversheet may further be different in different parts of the absorbent article. The "backsheet" refers to a material forming the outer cover of the absorbent article. The backsheet may be the same or different in different parts of the absorbent article. At least in the area of the absorbent medium, the backsheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The backsheet material may be breathable so as to allow vapour to escape from the absorbent material, while still preventing liquids from passing there through. Examples of breathable backsheet materials are porous polymeric films, nonwoven laminates of spunbond and meltblown layers and laminates of porous polymeric films and nonwoven materials.

The term "density" or "concentration" when referring to the absorbent material, in particular the SAP, of a layer, refers to the amount of the absorbent material divided by the surface area of the layer over which the absorbent material is spread out.

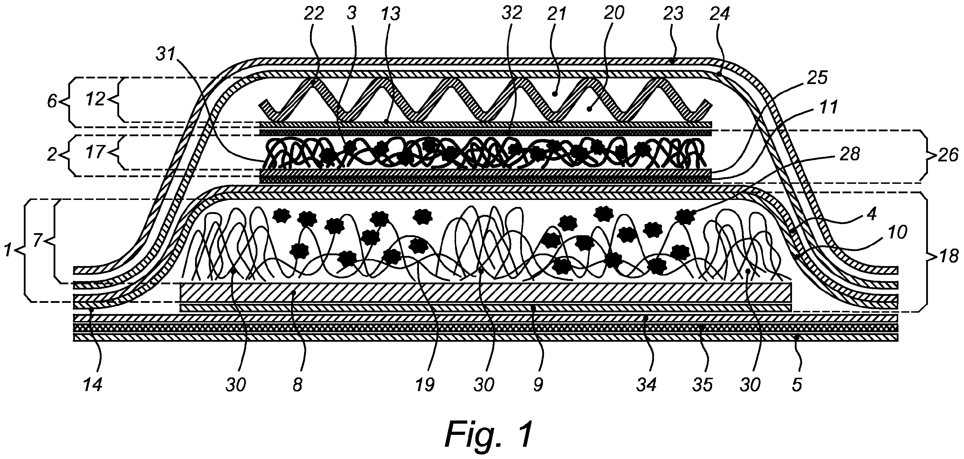

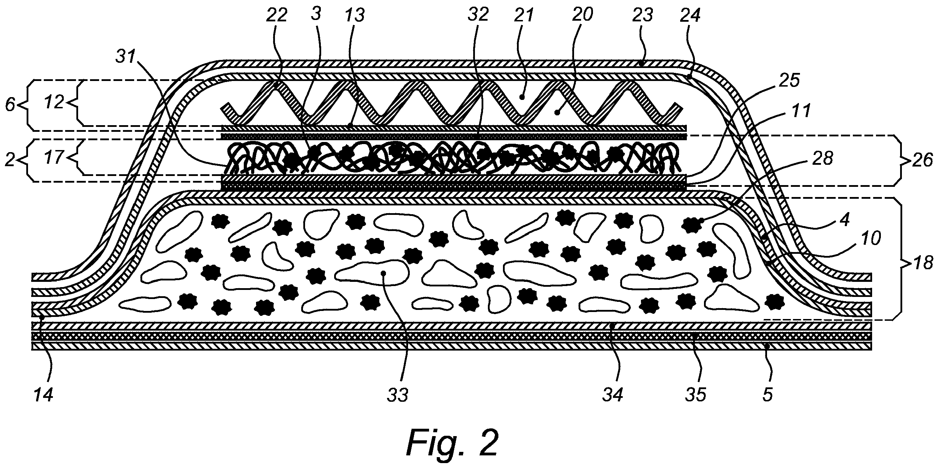

**FIG. 1** shows an embodiment of an absorbent article according to the present invention. **FIG. 1** is a general schematic cross-sectional view of the multi-layer structure of the absorbent material with a fluffless central absorbent layer (18) whereas **FIG. 2** is a general schematic cross-sectional view of a multi-layer structure of an absorbent material with a fluff pulp containing central absorbent layer (18).

**FIG. 2** does not show an embodiment of the invention. The inventors have found a way to provide an improved absorbent core and process for making such a core.

In particular, the present invention provides in a first aspect a multi-layer absorbent material comprising: a topsheet (23), a backsheet (5), a central absorbent layer (18) positioned between the topsheet (23) and the backsheet (5), said central absorbent layer (18) comprising superabsorbent polymer particles (28), an acquisition and distribution layer (6) positioned between the topsheet (23) and the central absorbent layer (18) for receiving fluids from the topsheet (23) and transferring the fluids for absorption by the central absorbent layer (18), and a booster absorbent layer (26) positioned between the acquisition and distribution layer (6) and the central absorbent layer (18) **(****Fig. 1****).**

The booster absorbent layer (26) comprises a nonwoven carrier (2) with superabsorbent polymer particles (3) whereby the booster absorbent layer is comprising a higher concentration of superabsorbent polymer particles than the central absorbent layer. The differential concentration of superabsorbent polymer particles between the two absorbent layers presents the advantage that the incoming fluids can be more efficiently absorbed than in prior art multi-layer absorbent materials, due to a two-fold process, firstly by the superabsorbent polymer particles (3) in said booster absorbent layer (26) and secondly by the superabsorbent polymer particles (28) in said central absorbent layer (18) **(****Fig. 1****).** Furthermore the booster layer can be placed at a region near a probable miction point. This allows to increase the absorbent capacity at this region without the need to provide an absorbent layer with location-dependent distribution of absorbent material, such as SAPs. Moreover, a higher concentration of SAP in the booster absorbent layer than in the central absorbent layer is present, as this leads to a larger absorption capacity by the booster absorbent layer, which is smaller than the central absorbent layer and preferably positioned closer to the middle of the absorbent article, i.e. further away from the edges, hereby reducing the probability of leakage.

Therefore, preferably the booster absorbent layer is disposed mainly near a possible miction point, more preferably at least 50%, even more preferably at least 55%, still more preferably at least 60%, yet more preferably at least 65% of the booster absorbent layer is disposed in the front half of the absorbent article. The booster absorbent layer increases the absorbency at the region near possible miction points, which reduces the probability of leakage of fluids which could not be absorbed fast enough in prior art absorbent articles. Furthermore, fluids which pass through the booster absorbent layer without directly being absorbed, can be absorbed by the central absorbent layer. The above described situation occurs frequently as e.g. urine discharges usually result in a large amount of fluids being discharged in a small area and in a short period.

A further advantage of the present invention, is the ease of producing such an absorbent article whereby the absorbent capacity can be increased near a possible miction point, in particular by the presence of the at least two absorbent layers. The use of two layers, e.g. instead of one layer with differential absorption capacity, allows to produce absorbent articles of different types and of different sizes using the same components, methods and/or apparatuses.

In a preferred embodiment, the multi-layer absorbent material is comprising the central absorbent layer (18) as a first absorbent core layer of a first size, and at least one additional booster absorbent layer (26) overlying the first absorbent core layer and being of a different size than the first absorbent core layer thereby forming an absorbent multi-layer core. The booster absorbent layer is comprising a maximum length of between 5 cm and 50 cm, more preferably between 10 cm and 40 cm, yet more preferably between 20 cm and 30 cm and a maximum width of between 2.5 cm and 30 cm, more preferably between 5 cm and 20 cm, still more preferably between 7.5 cm and 15 cm. The central absorbent layer is comprising a maximum length of between 5 cm and 80 cm, more preferably between 15 cm and 65 cm, yet more preferably between 30 cm and 50 cm and a maximum width of between 2.5 cm and 50 cm, more preferably between 5 cm and 35 cm, still more preferably between 10 cm and 20 cm.

In a preferred embodiment, the booster absorbent layer (26) is comprising an amount of superabsorbent polymer particles (3) which is between 1 g and 30 g, more preferably between 2 g and 20 g, yet more preferably between 3 g and 10 g.

In a preferred embodiment, the central absorbent layer (18) is comprising an amount of superabsorbent polymer particles (28) which is between 1 g and 30 g, more preferably between 2 g and 20 g, yet more preferably between 3 g and 10 g.

In a preferred embodiment, the central and/or booster absorbent layer comprise superabsorbent particles which are distributed according to a pattern, preferably a pattern comprising areas (30) which are essentially free from SAPs, such as a clustered pattern.

In a preferred embodiment, the central absorbent layer (18) is comprising a fluffless top layer (7) and superabsorbent polymer particles (28) **(****Fig.1****).**

In the various aspects of the invention, the central absorbent layer (18) comprises a fluffless top layer (7). As in the previous aspects of the invention, these materials will be configured to provide maximum permeability while maintaining enough capillary tension to control the movement of the liquid and not allow leakage to occur. For example, the central absorbent layer (18) of the present invention incorporates nonwoven materials with superabsorbent polymer particles in the fluffless top layer (7).

In a preferred embodiment, the central absorbent layer (18) is comprising superabsorbent polymer particles (28) that are immobilized in a nonwoven carrier (1) comprising a top layer (7) comprising staple fibers (19) penetrable by the superabsorbent polymer particles (28) and a nonwoven bottom layer (8), bonded, preferably mechanically bonded, more preferably hydroentangled to the top layer (7). The superabsorbent polymer particles (28) are trapped in between the staple fibers (19) which limits their movement in the longitudinal and the transversal directions (x-y plane) as well as in the z-directionof the absorbent article. Their distribution is limited by the nonwoven bottom layer (8). The average size of the pores of the nonwoven bottom layer (8) is smaller than the average particle size of the superabsorbent polymer particles (28), preventing the particles from falling through the material. The sizes of superabsorbent polymer particles (28) depend on the exact application, but for absorbent articles such as diaper, the particle size is mainly ranging between 100 to 800 µm, preferably between 300 to 600 µm, more preferably between 400 and 500 µm. Therefore, in a preferred embodiment, the nonwoven bottom layer (8) has a pore size smaller than 300 µm, more preferably smaller than 100 *µm* thereby retaining the superabsorbent polymer particles (28). The central absorbent layer (18) is preferably attached by thermoplastic adhesive (11) to the booster absorbent layer (26). Note that the central absorbent layer may be provided with a core wrap (4, 34) as indicated in figs. 1 and 2.

The central absorbent layer (18) is preferably wrapped between two layers of nonwoven (4, 34), the so-called core wrap. Or equivalently, the central absorbent layer preferably comprises a core wrap, which preferably comprise two layers of nonwoven (4, 34). The lower core wrap (34) can be attached to the bottom layer (8) of the nonwoven carrier (1) by thermoplastic adhesive (9). The upper core wrap (4) can be attached to the top surface of the nonwoven carrier (1) by thermoplastic adhesive (10). The superabsorbent polymer particles (28) can be partly in contact with the adhesive coating (10) on the upper core wrap (4).

In a preferred embodiment, the widths of the core wrap nonwovens exceed the width of the nonwoven carrier (1), so that the core wrap nonwovens can be bonded with each other along the side edges by thermoplastic adhesive (14). The core-wrapped central absorbent layer (18) can be attached to the back sheet (5), e.g. by a thermoplastic adhesive layer (35).

The booster absorbent layer (26) is comprising superabsorbent polymer particles (3) that are immobilized in a nonwoven carrier (2) comprising a top layer (17) consisting of staple fibers (31) penetrable by the superabsorbent polymer particles (3) and a nonwoven bottom layer (25), hydroentangled to the top layer (17) **(****Fig. 1****).**

The superabsorbent polymer particles (3) are trapped in between the staple fibers (31) which limits their movement in the longitudinal and the transversal directions (x-y plane) as well as in the z- of the absorbent article. Their distribution is limited by the nonwoven bottom layer (25). The average size of the pores of the nonwoven bottom layer (25) is smaller than the average particle size of the superabsorbent polymer particles (3), preventing the particles from falling through the material. The sizes of superabsorbent polymer particles (3) depend on the exact application, but for absorbent articles such as diaper, the particle size is mainly ranging between 100 to 800 µm, preferably between 300 to 600 µm, more preferably between 400 and 500 µm. Therefore, in a preferred embodiment, the nonwoven bottom layer (25) has a pore size smaller than 300 µm, more preferably smaller than 100 µm thereby retaining the superabsorbent polymer particles (3). The booster absorbent layer (26) has the capacity to receive and either absorb or temporarily hold the fluids in proximity to the central absorbent layer (18), avoiding leakage to occur. On top of the booster absorbent layer (26), an acquisition and distribution layer (6) is placed which is preferably attached by thermoplastic adhesive (32) to the booster absorbent layer (26). Preferably, the acquisition and distribution layer (6) is based upon Nonwovens Innovation & Research Institute's proprietary Hydrospace technology as described in EP 1644564A1 **(****Fig. 1****).**

Therefore, preferably, the ADL according to the present invention comprises a thin, 3D nonwoven spacer fabric with discrete, channel-like voids or cells within the fabric cross-section, which relies on fluid forces rather than conventional mechanical methods to periodically interconnect, e.g. hydro-entangle, fibres from at least two web structures which are separated by a spacer system during their production. Also preferably, the ADL comprises a nonwoven fabric, the fabric comprising at least two separate but interconnected layers, each of the layers being provided with discrete interconnections so as to provide discrete voids between the two layers of fabric. The shape of the voids may vary. However, preferentially, the voids comprise a channel and/or a tube, e. g. a plurality of channels and/or tubes within the structure of the fabric. The channels and/or tubes may be substantially cylindrical in shape. However, it will be understood by one skilled in the art that the size and/or shape of the voids may be influenced by the choice of the spacer material. Similarly, the size of the voids may vary, depending, inter alia, on the nature of the use of the nonwoven fabric. However, preferentially, the channels and/or tubes are such that they comprise a diameter in the range of from 0.2 mm to 8.5 mm, more preferably from 0.5 mm to 6 mm, yet more preferably from 1 mm to 5 mm, e.g. 2 mm, 3 mm, 4 mm or any value therebetween. The acquisition and distribution layer (6) can preferably be attached by thermoplastic adhesive (32) to the underneath booster absorbent layer (26). The multi-layer absorbent material being covered by a nonwoven topsheet (23) attached to the components underneath with a layer of thermoplastic adhesive (24). This is particularly preferred for absorbent articles comprising such an ADL or if such an ADL is to be used in an absorbent article. Fluids are substantially guided in the longitudinal direction through the channels (21) and are further collected in the tubes (20) by e.g. capillary absorption that allows for the fluids to flow along the tubes (20). This promotes removal of a stationary portion of the fluids present on the nonwoven topsheet (23) and results in a better distribution of the fluids through the acquisition and distribution layer (6), before penetrating first the booster absorbent layer (26) and afterwards the underneath absorbent core (18).

In a preferred embodiment, the acquisition and distribution layer (6) is comprising a top layer (12) comprising staple fibers (22) and a, preferably essentially flat, non woven bottom layer (13), the acquisition and distribution layer (6) comprising an undulated structure. In a preferred embodiment, said top-layer (12) comprises corrugations which at least partially define said undulated channel structure. In a preferred embodiment, the ADL (6) comprises a maximum length of between 5 cm and 50 cm, more preferably between 10 cm and 40 cm, yet more preferably between 20 cm and 30 cm, and a maximum width of between 2.5 cm and 30 cm, more preferably between 5 cm and 20 cm, still more preferably between 7.5 cm and 15 cm. In a preferred embodiment, the undulated structure comprises two neighboring tubes (20) separated by a channel (21) and/or two neighboring channels (21) separated by a tube, whereby said two neighboring tubes and/or channels comprise a separating distance of between 3 mm and 20 mm, preferably between 5 mm and 15 mm, more preferably between 8 mm and 13 mm, still more preferably 9, 10, 11 or 12 mm, the distance being measured between the center of the neighboring tubes or channels (21), to improve fluids distribution, and a nonwoven bottom layer (13) **(****Fig.** 1).

In a preferred embodiment, the acquisition and distribution layer (6) and the booster absorbent layer (26) are about the same size, preferably the length and/or width of the ADL differs from the length and/or width of the booster absorbent layer by less than 50%, more preferably by less than 40%, still more preferably by less than 30%, yet more preferably by less than 20%, even more preferably by less than 10%, still even more preferably by less than 5%.

In a second aspect, the invention provides an absorbent article comprising a multi-layer absorbent material according to an embodiment of the invention.

In a third aspect, the invention provides a process for manufacturing a multi-layer absorbent material according to an embodiment of the invention, comprising the steps of:
a) providing a topsheet (23) and a backsheet (5);
b) providing an acquisition and distribution layer (6);
c) providing an absorbent multi-layer core by:
   c1) providing a booster absorbent layer (26);
   c2) providing a central absorbent layer (18); and
d) disposing the absorbent multi-layer core at least partially between the top sheet (23) and the back sheet (5) and disposing the booster absorbent layer (26) between the acquisition and distribution layer (6) and the central absorbent layer (18),
thereby providing the multi-layer absorbent material.

In a preferred embodiment, the acquisition and distribution layer (6) and the booster absorbent layer (26) are placed mainly within the front half of the central absorbent layer (18) to distribute and absorb fluids, preferably at least 50%, more preferably at least 55%, still more preferably at least 60%, yet more preferably at least 65%, still even more preferably at least 70% of the ADL and/or the booster absorbent layer are disposed within the front half of the central absorbent layer.

In an optional embodiment, the central absorbent layer (18) is comprising a nonwoven carrier and a top layer comprising staple fibers, wherein preferably the method further comprises the step of treating the top layer and the nonwoven carrier with a surfactant.

The acquisition distribution layer (6), the nonwoven carrier (2), and the nonwoven carrier (1) may be treated with a surfactant. The acquisition distribution layer (6), the nonwoven carrier (2), and the nonwoven carrier (1) can preferably be rendered hydrophilic through the use of a post treatment application of a surfactant. The hydrophilic characteristics of the acquisition distribution layer (6), the nonwoven carrier (2), and the nonwoven carrier (1) improve absorbent articles such as baby diapers and adult incontinence diapers by improving absorbency.

In a final aspect, the invention provides use of a multi-layer absorbent material according to an embodiment of the invention in an absorbent article and/or for manufacturing an absorbent article.

Although the present invention has been described with reference to preferred embodiments thereof, many modifications and alterations may be made by a person having ordinary skill in the art without departing from the scope of this invention which is defined by the appended claims.

## Claims

1. A multi-layer absorbent material comprising: a topsheet (23), a backsheet (5), a central absorbent layer (18) positioned between the topsheet (23) and the backsheet (5), said central absorbent layer (18) comprising superabsorbent polymer particles (28), an acquisition and distribution layer (6) positioned between the topsheet (23) and the central absorbent layer (18) for receiving fluids from the topsheet (23) and transferring the fluids for absorption to the central absorbent layer (18), and a booster absorbent layer (26) positioned between the acquisition and distribution layer (6) and the central absorbent layer (18), the booster absorbent layer (26) comprising a nonwoven carrier (2) with superabsorbent polymer particles (3), whereby the booster absorbent layer (26) comprises a higher concentration of superabsorbent polymer particles than the central absorbent layer (18), the concentration of superabsorbent polymer particles on a layer being defined as the amount of the absorbent material divided by the surface area of the layer over which the absorbent material is spread out, whereby the booster absorbent layer (26) comprises a smaller surface area than the central absorbent layer (18), **characterized in that** the booster absorbent layer (26) is comprising superabsorbent polymer particles (3) that are immobilized in a nonwoven carrier (2) comprising a top layer (17) consisting of staple fibers (31) penetrable by the superabsorbent polymer particles (3) and a nonwoven bottom layer (25) mechanically bonded by hydro-entanglement to the top layer (17), wherein the nonwoven bottom layer (25) is porous with an average pore size smaller than the average size of the superabsorbent polymer particles (3), and wherein the central absorbent layer (18) is comprising a fluffless absorbent layer and superabsorbent polymer particles.

2. The multi-layer absorbent material according to claim 1, wherein the booster absorbent layer (26) and/or the central absorbent layer (18) is comprising an amount of superabsorbent polymer particles which is between 1 g and 30 g, more preferably between 2 g and 20 g, yet more preferably between 3 g and 10 9.

3. The multi-layer absorbent material according to any of the previous claims, wherein the central absorbent layer (18) is comprising superabsorbent polymer particles (28) that are immobilized in a nonwoven carrier (1) comprising a top layer (7) comprising staple fibers (19) penetrable by the superabsorbent polymer particles (28) and a nonwoven bottom layer (8), mechanically bonded by hydro-entanglement to the top layer (7); wherein the nonwoven bottom layer (8) is porous with an average pore size smaller than the average size of the superabsorbent polymer particles (28).

4. The multi-layer absorbent material according to any of the previous claims, wherein the acquisition and distribution layer (6) is comprising a top layer (12) comprising staple fibers (22) and a, preferably essentially flat, nonwoven bottom layer (13), the acquisition and distribution layer (6) comprising an undulated structure.

5. The multi-layer absorbent material according to any of the previous claims, wherein the acquisition and distribution layer (6) and the booster absorbent layer (26) each comprise a length and/or a width, whereby the length and/or width of the ADL differs from the length and/or width of the booster absorbent layer by less than 50%, more preferably by less than 40%, still more preferably by less than 30%, yet more preferably by less than 20%, even more preferably by less than 10%, still even more preferably by less than 5%.

6. An absorbent article comprising a multi-layer absorbent material according to any of claims 1 to5.

7. Process for manufacturing a multi-layer absorbent material according to any of claims 1 to 5, comprising the steps of:
a) providing a topsheet (23) and a backsheet (5);
b) providing an acquisition and distribution layer (6);
c) providing an absorbent multi-layer core by:
c1) providing a booster absorbent layer (26);
c2) providing a central absorbent layer (18);
d) disposing the absorbent multi-layer core between the topsheet (23) and the backsheet (5) and disposing the booster absorbent layer (26) between the acquisition and distribution layer (6) and the central absorbent layer (18),
thereby providing the multi-layer absorbent material.

8. Process according to claim 7, wherein at least 50%, more preferably at least 55%, still more preferably at least 60%, yet more preferably at least 65%, still even more preferably at least 70% of the ADL (6) and/or the booster absorbent layer (26) are disposed within the front half of the central absorbent layer (18).

9. Process according to any of the claims 7 to 8, wherein the central absorbent layer (18) is comprising a nonwoven carrier (1) and a top layer (7) comprising staple fibers, the method further comprising the step of treating the top layer and the nonwoven carrier with a surfactant.

10. Use of a multi-layer absorbent material according to any of claims 1 to 5 for manufacturing an absorbent article.

## Patentansprüche

1. Ein mehrschichtiges absorbierendes Material, umfassend: ein Oberblatt (23), ein Rückblatt (5), eine zentrale absorbierende Schicht (18), die zwischen dem Oberblatt (23) und dem Rückblatt (5) positioniert ist, die zentrale absorbierende Schicht umfassend superabsorbierende Polymerpartikel (28), eine Aufnahme- und Verteilungsschicht (6), die zwischen dem Oberblatt (23) und der zentralen absorbierenden Schicht (18) positioniert ist, zum Aufnahme von Flüssigkeiten von dem Oberblatt (23) und zur Übertragung der Flüssigkeiten zur Absorption zu der zentralen absorbierenden Schicht (18), und eine absorbierende Boosterschicht (26), die zwischen der Aufnahme- und Verteilungsschicht (6) und der zentralen absorbierenden Schicht (18) positioniert ist, wobei die absorbierende Boosterschicht (26) einen Vliesstoffträger (2) mit superabsorbierenden Polymerpartikeln (3) umfasst, wobei die absorbierende Boosterschicht (26) eine höhere Konzentration von superabsorbierenden Polymerpartikeln als die zentrale absorbierende Schicht (18) umfasst, wobei die Konzentration von superabsorbierenden Polymerpartikeln auf einer Schicht als die Menge des absorbierenden Materials geteilt durch die Fläche der Schicht, über die das absorbierende Material ausgestrichen ist, definiert ist, wobei die absorbierende Boosterschicht (26) eine kleinere Fläche als die zentrale absorbierende Schicht (18) umfasst, **dadurch gekennzeichnet, dass** die absorbierende Boosterschicht (26) superabsorbierende Polymerpartikel (3) umfasst, die in einem Vliesstoffträger (2) immobilisiert sind, der eine Oberschicht (17) aus Stapelfasern (31) umfasst, die durch die superabsorbierenden Polymerpartikeln (3) durchdringbar ist und eine untere Vliesstoffschicht (25), die mechanisch durch Wasserstrahlverwirbelung zu der Oberschicht (17) verbunden ist, wobei die untere Vliesstoffschicht (25) porös ist mit einer mittleren Porengröße, die kleiner ist als die mittlere Größe der superabsorbierenden Polymerpartikel (3), und wobei die zentrale absorbierende Schicht (18) eine fusselfreie absorbierende Schicht und superabsorbierende Polymerpartikel umfasst.

2. Das mehrschichtige absorbierende Material nach Anspruch 1, wobei die absorbierende Boosterschicht (26) und/oder die zentrale absorbierende Schicht (18) eine Menge an superabsorbierenden Polymerpartikeln umfasst, die zwischen 1 g und 30 g, bevorzugt zwischen 2 g und 20 g, noch bevorzugter zwischen 3 g und 10 g liegt.

3. Das mehrschichtige absorbierende Material nach einem der vorhergehenden Ansprüche, wobei die zentrale absorbierende Schicht (20) superabsorbierende Polymerpartikel (28) umfasst, die in einem Vliesstoffträger (1) immobilisiert sind, der eine Oberschicht (7) umfassend Stapelfasern (19) umfasst, die durch die superabsorbierenden Polymerpartikeln (28) durchdringbar ist und eine untere Vliesstoffschicht (8), die mechanisch durch Wasserstrahlverwirbelung zu der Oberschicht (7) verbunden ist, wobei die untere Vliesstoffschicht (8) porös ist mit einer mittleren Porengröße, die kleiner ist als die mittlere Größe der superabsorbierenden Polymerpartikel (28).

4. Das mehrschichtige absorbierende Material nach einem der vorhergehenden Ansprüche, wobei die Aufnahme- und Verteilungsschicht (6) eine Oberschicht (12) umfassend Stapelfasern (22) umfasst und eine, vorzugsweise im Wesentlichen flache, untere Vliesstoffschicht (13), wobei die Aufnahme- und Verteilungsschicht (6) eine gewellte Struktur umfasst.

5. Das mehrschichtige absorbierende Material nach einem der vorhergehenden Ansprüche, wobei die Aufnahme- und Verteilungsschicht (6) und die absorbierende Boosterschicht (26) jeweils eine Länge und/oder eine Breite umfassen, wobei die Länge und/oder die Breite der ADL sich von der Länge und/oder Breite der absorbierenden Boosterschicht unterscheidet um weniger als 50%, bevorzugter um weniger als 40%, mehr bevorzugt um weniger als 30%, noch mehr bevorzugt um weniger als 20%, noch stärker bevorzugt um weniger als 10%, noch besonders bevorzugt um weniger als 5%.

6. Ein absorbierender Artikel umfassend ein mehrschichtiges absorbierendes Material nach einem der Ansprüche 1 bis 5.

7. Verfahren zur Herstellung eines mehrschichtigen absorbierenden Materials nach einem der Ansprüche 1 bis 5, umfassend die Schritte von:
a) Bereitstellen eines Oberblattes (23) und eines Rückblattes (5);
b) Bereitstellen einer Aufnahme- und Verteilungsschicht (6);
c) Bereitstellen eines absorbierenden mehrschichtigen Kerns durch das:
c1) Bereitstellen einer absorbierenden Boosterschicht (26);
c2) Bereitstellen einer zentralen absorbierenden Schicht (18);
d) Anordnen des absorbierenden mehrschichtigen Kerns zwischen dem Oberblatt (23) und dem Rückblatt (5) und das Anordnen der absorbierenden Boosterschicht (26) zwischen der Aufnahme- und Verteilungsschicht (6) und der zentralen absorbierenden Schicht (18),
wodurch das mehrschichtige absorbierende Material bereitgestellt wird.

8. Verfahren nach Anspruch 7, wobei mindestens 50%, mehr bevorzugt mindestens 55%, noch mehr bevorzugt mindestens 60%, noch stärker bevorzugt mindestens 65%, noch besonders bevorzugt mindestens 70% der ADL (6) und/oder der absorbierenden Boosterschicht (26) innerhalb der vorderen Hälfte der zentralen absorbierenden Schicht (18) angeordnet sind.

9. Verfahren nach einem der Ansprüche 7 bis 8, wobei die zentrale absorbierende Schicht (18) einen Vliesstoffträger (1) und eine Oberschicht (7) umfasst, umfassend Stapelfasern, wobei das Verfahren ferner den Schritt des Behandelns der Oberschicht und des Vliesstoffträgers mit einem Tensid umfasst.

10. Verwendung eines mehrschichtigen absorbierenden Materials nach einem der Ansprüche 1 bis 5 zur Herstellung eines absorbierenden Artikels.

## Revendications

1. Un matériau absorbant multicouche comprenant: une feuille supérieure (23), une feuille arrière (5), une couche absorbante centrale (18) positionnée entre la feuille supérieure (23) et la feuille inférieure (5), ladite couche absorbante centrale (18) comprenant des particules de polymère superabsorbantes (28), une couche d'acquisition et de distribution (6) positionnée entre la feuille supérieure (23) et la couche absorbante centrale (18) pour recevoir des fluides de la feuille supérieure (23) et transférer les fluides pour absorption à la couche absorbante centrale (18), et une couche absorbante booster (26) positionnée entre la couche d'acquisition et de distribution (6) et la couche absorbante centrale (18), la couche absorbante booster (26) comprenant un support non-tissé (2) avec des particules de polymère superabsorbantes (3), dans lequel la couche absorbante booster (26) comprend une plus grande concentration de particules de polymère superabsorbantes que la couche absorbante centrale (18), la concentration des particules de polymère superabsorbantes sur une couche étant définie comme la quantité de matériau absorbant divisée par la surface de la couche sur laquelle le matériau absorbant est étalé, dans lequel la couche absorbante booster (26) comprend une surface plus petite que celle de la couche absorbante centrale (18), **caractérisé en ce que** la couche absorbante booster (26) comprend des particules de polymère superabsorbantes (3) qui sont immobilisées dans un support non-tissé (2) comprenant une couche supérieure (17) constituée de fibres discontinues (31) pénétrable par les particules de polymère superabsorbantes (3) et une couche inférieure non-tissée (25) liée mécaniquement par hydro-enchevêtrement à la couche supérieure (17), dans lequel la couche inférieure non-tissée (25) est poreuse avec une taille de pore moyenne plus petite que la taille moyenne des particules de polymère superabsorbantes (3), et dans lequel la couche absorbante centrale (18) comprend une couche non-pelucheuse absorbante et des particules de polymère superabsorbantes.

2. Le matériau absorbant multicouche selon la revendication 1, dans lequel la couche absorbante booster (26) et/ou la couche absorbante centrale (18) comprend une quantité de particules de polymère superabsorbantes qui est entre 1 g et 30 g, plus préférentiellement entre 2 g et 20 g, encore plus préférentiellement entre 3 g et 10 g.

3. Le matériau absorbant multicouche selon l'une quelconque des revendications précédentes, dans lequel la couche absorbante centrale (18) comprend des particules de polymère superabsorbantes (28) qui sont immobilisées dans un support non-tissé (1) comprenant une couche supérieure (7) comprenant des fibres discontinues (19) pénétrable par les particules de polymère superabsorbantes (28) et une couche inférieure non-tissée (8), liée mécaniquement par hydro-enchevêtrement à la couche supérieure (7); dans lequel la couche inférieure non-tissée (8) est poreuse avec une taille de pore moyenne plus petite que la taille moyenne des particules de polymère superabsorbantes (28).

4. Le matériau absorbant multicouche selon l'une quelconque des revendications précédentes, dans lequel la couche d'acquisition et de distribution (6) comprend une couche supérieure (12) comprenant des fibres discontinues (22) et une couche inférieure non-tissée (13), de préférence essentiellement plate, la couche d'acquisition et de distribution (6) comprenant une structure ondulée.

5. Le matériau absorbant multicouche selon l'une quelconque des revendications précédentes, dans lequel la couche d'acquisition et de distribution (6) et la couche absorbante booster (26) comprennent chacune une longueur et/ou une largeur, dans lequel la longueur et/ou largeur de l'ADL est différente de la longueur et/ou largeur de la couche absorbante booster de moins de 50%, plus préférablement de moins de 40%, encore plus préférablement de moins de 30%, encore plus préférablement de moins de 20%, encore plus de préférence de moins de 10%, encore plus préférablement de moins de 5%.

6. Un article absorbant comprenant un matériau absorbant multicouche selon l'une quelconque des revendications 1 à 5.

7. Processus pour fabriquer un matériau absorbant multicouche selon l'une quelconque des revendications 1 à 5, comprenant les étapes de:
a) fournir une feuille supérieure (23) et une feuille arrière (5);
b) fournir une couche d'acquisition et de distribution (6);
c) fournir un noyau absorbant multicouche par:
c1) fournir une couche absorbante booster (26);
c2) fournir une couche absorbante centrale (18);
d) disposer le noyau absorbant multicouche entre la feuille supérieure (23) et la feuille arrière (5) et disposer la couche absorbante booster (26) entre la couche d'acquisition et de distribution (6) et la couche absorbante centrale (18),
fournissant ainsi le matériau absorbant multicouche.

8. Processus selon la revendication 7, dans lequel au moins 50%, plus préférablement au moins 55%, encore plus préférablement au moins 60%, encore plus préférablement au moins 65%, encore plus préférablement au moins 70% de l'ADL (6) et/ou de la couche absorbante booster (26) sont disposés dans la moitié avant de la couche absorbante centrale (18).

9. Processus selon l'une quelconque des revendications 7 à 8, dans lequel la couche absorbante centrale (18) comprend un support non-tissé (1) et une couche supérieure (7) comprenant des fibres discontinues, le processus comprenant en outre l'étape de traiter la couche supérieure et le support non-tissé avec un agent tensioactif.

10. Utilisation d'un matériau absorbant multicouche selon l'une quelconque des revendications 1 à 5 pour fabriquer un article absorbant.
